# EUROPÄISCHE PATENTANMELDUNG

(11) **EP 4 349 288 A1**
(43) Veröffentlichungstag der Anmeldung: **10.04.2024**
(21) Anmeldenummer: 22200327.9
(22) Anmeldetag: 07.10.2022
(51) Int. Cl.: A61B 18/14, A61B 18/00

(54) **ABLATIONSSONDE MIT INNERER KÜHLUNG**

(71) Anmelder: Erbe Elektromedizin GmbH, 72072 Tübingen (DE)
(72) Erfinder: ANDEL, Hanna, 72116 Moessingen (DE); ADLER, Marcus, 72393 Burladingen (DE); KARCHER, Felix, 72072 Tuebingen (DE)
(74) Vertreter: Rüger Abel Patentanwälte PartGmbB

(57) **Zusammenfassung**

Eine erfindungsgemäße Ablationssonde (10) weist wenigstens zwei Elektroden (15, 16) auf, die auf einem Schlauch (11) gehalten sind. Endseitig ist die Ablationssonde (10) mit einem Verschlussstück (17) versehen, von dem ausgehend sich ein Draht (28) über die gesamte Länge des Schlauchs (12) durch diesen erstreckt. An dem Draht (28) ist eine Fluidzulieferleitung (29) befestigt, die seitliche Öffnungen (32, 33) zur Kühlung der Elektroden (15, 16) aufweist und die endseitig geschlossen ist. Durch die Fixierung der Fluidzulieferleitung (29) an dem Draht (28) wird eine axiale Ausrichtung der Öffnungen (32, 33) relativ zu den Elektroden (15, 16) erreicht und eine Fehlpositionierung vermieden, die ansonsten zur Verungleichmäßigung der Kühlung der Elektroden (15, 16) führen könnte.

## Beschreibung

Die Erfindung betrifft eine Ablationssonde insbesondere zur HF-Ablation von biologischem Gewebe.

Sonden zur HF-Ablation dienen beispielsweise der Devitalisierung von Gewebeabschnitten, zum Beispiel der Devitalisierung von in Gewebe (z.B. Lunge oder Leber) eingebetteten Tumoren oder dergleichen.

Die EP 3 788 974 A1 veranschaulicht eine Ablationssonde der genannten Bauart mit zwei auf einem schlauchartigen Grundkörper gehaltenen Elektroden, die mit dem Ausgang eines elektrischen Generators zu verbinden sind. Die Elektroden sind in axialem Abstand zueinander auf dem distalen Ende des schlauchartigen Grundkörpers angeordnet, um, wenn sie in Gewebe eingestochen sind, Strom durch dieses zu schicken und dadurch zu erwärmen.

Es hat sich als zweckmäßig herausgestellt, die Elektroden zu kühlen, um einen Austrocknung des mit den Elektroden in Berührung stehenden Gewebes zu vermeiden. Dazu wird das von dem Schlauch umschlossene Lumen mit einem Kältemittel versorgt, sodass die Elektrodentemperatur in Grenzen gehalten wird.

Dieses Prinzip verwirklichen auch Sonden, wie sie in der EP 1 181 896 A1, der WO 2012/012869 A1 oder der US 2005/0010201 A1 veranschaulicht sind. Weiteren Stand der Technik offenbaren die EP 3 323 366 A1, die EP 3 769 706 A1, die US 6,106,524, die WO 99/08633 A, die EP 1 432 360 B1, die EP 2 768 563 B1, die EP 0 754 075 B1, die EP 3 763 314 B1, die EP 3 437 579 B1 und die WO 94/11059 A. Außerdem ist aus der DE 10 2008 024 946 A1 eine Kryosonde bekannt, die eine Kryofluidkapillare mit seitlicher Austrittsöffnung aufweist.

Bei der Behandlung von Gewebe sollen die Elektroden gekühlt werden, damit das unmittelbar in Kontakt mit den Elektroden stehende Gewebe nicht durch Austrocknung hochohmig wird, sondern feucht und somit niederohmig bleibt. Deswegen wird das zur Kühlung dienende Kältemittel an dem distalen Ende des Instruments freigesetzt. Dazu ist in dem Lumen des Schlauchs eine Fluidzuführkapillare angeordnet, die an ihrem distalen Ende mindestens eine Öffnung aufweist, über die das kühlende Fluid austritt und das distale Ende der Sonde und somit auch die Elektroden kühlt. Diese Bauart ist insbesondere in der EP 3 788 974 A1 beschrieben.

Die WO 2012/012869 offenbart einen Herzkatheter mit einer Fluidzuführleitung, die in dem von dem Katheter umschlossenen Lumen angeordnet ist und in der Nähe seines distalen Endes mehrere laterale Ausströmöffnungen aufweist. Diese dienen der Kühlmittelzufuhr zu dem distalen Ende des Katheters.

Die US 2005/0010201 A1 offenbart eine Sonde zur Kältebehandlung von biologischem Gewebe. Die Sonde besteht im Wesentlichen aus einem Schlauchkörper, an dessen distalen Ende mehrere die Schlauchwand durchsetzende wärmeleitenden Elemente angeordnet sind. In dem Lumen des Schlauchs ist in einer ersten Variante ein Fluidzufuhrschlauch angeordnet, der mehrere seitliche Austrittsöffnungen aufweist, die jeweils einen Fluidstrom auf das jeweilige wärmeleitende Element richten. In einer anderen Variante sind in dem Lumen der Sonde mehrere Fluidkapillaren angeordnet, deren jeweilige distale Austrittsöffnung jeweils einem wärmeleitenden Element zugeordnet ist.

Um das Einstechen einer Sonde in biologisches Gewebe zu erleichtern, ist es außerdem bekannt, das distale Ende der Sonde außen mit einer beispielsweise bügelförmigen Elektrode zu versehen, die mit einer elektrischen Stromzufuhr verbunden ist und, wenn sie aktiviert wird, in das Gewebe schneidend wirksam wird.

Es wird zunehmend gewünscht, derartige Ablationssonden ohne Einschränkung ihrer Funktionalität hinsichtlich der Größe der zu behandelnden Gewebepartien möglichst schlank, d.h. mit geringem Durchmesser bereitzustellen. Es werden dabei Durchmesser von unter 2,5 mm angestrebt, wodurch auch die zur Verfügung stehende Elektrodenfläche entsprechend geringer wird. Andererseits steigt dadurch die Stromdichte an der Elektrodenoberfläche und somit der Kühlbedarf der Elektroden an.

Davon ausgehend, ist es Aufgabe der Erfindung, eine Ablationssonde mit innerer Kühlung bereitzustellen, wobei die Erfindung insbesondere die weiteren Verringerung des Sondendurchmessers und andererseits die geforderte Qualität der Ablationshandlung sicherstellen soll.

Diese Aufgabe wird mit der Ablationssonde nach Anspruch 1 gelöst:

Die Ablationssonde weist einen flexiblen Schlauch auf, auf dem mindestens eine oder auch zwei oder mehrere voneinander in Axialrichtung des Schlauchs distanzierten Elektroden angeordnet sind. Diese sind auf dem oder nahe zu dem distalen Ende des Schlauchs angeordnet. Vorzugsweise erstrecken sich die Elektroden dabei um den gesamten Umfang des Schlauchs herum sowie über eine (zwischen ihrem distalen und ihrem proximalen Ende zu messende) axialen Länge, die vorzugsweise ein Mehrfaches ihres Durchmessers beträgt. Die Elektroden sind vorzugsweise flexibel ausgebildet, so dass sie engen seitlichen Krümmungen folgen können. Sie können dazu nach Art von Schraubenfedern ausgebildet sein. Dadurch weisen sie in Axialrichtung eine gegenüber einer metallischen Hülse reduzierte elektrische und thermische Leitfähigkeit auf.

Die Elektroden sind an elektrische Leitungen angeschlossen, die sich von der jeweiligen Elektrode bis zu dem proximalen Ende des Schlauchs erstrecken und dort über geeignete Anschlussmittel mit einem speisenden Generator verbindbar sind. Der speisende Generator ist vorzugsweise darauf eingerichtet, eine hochfrequente Wechselspannung bereitzustellen und somit hochfrequenten Strom zu geben.

Der Schlauch umschließt ein Lumen, das sich von seinem proximalen Ende bis zu seinem distalen Ende erstreckt und dort abgeschlossen ist. Dazu kann zum Beispiel ein Verschlussstück vorgesehen sein, das mit dem Schlauch fest verbunden ist und dessen Lumen verschließt. In dem Lumen ist eine Fluidlieferleitung angeordnet, die sich von dem proximalen Ende des Schlauchs in distaler Richtung bis zu den Elektroden erstreckt. An dem proximalen Ende kann die Fluidlieferleitung mit einer geeigneten Anschlusseinrichtung versehen sein, um mit einer zum Beispiel in dem speisenden Gerät angeordneten Kryofluidquelle verbunden zu werden. Das speisende Gerät kann somit die Kryofluidquelle zur Kühlung der Elektroden und den Generator zur Bestromung der Elektroden in einem Gerät vereinen. Getrennte Ausführungen sind ebenfalls möglich. Solche separaten Geräte können über eine geeignete Schnittstelle, z.B. einen BUS zu einem Gerätesystem verbunden werden.

Die Fluidlieferleitung ist vorzugsweise endseitig geschlossen, wobei sie im Bereich der Elektroden jeweils mindestens eine seitliche Öffnung aufweist. Auf diese Weise wird das Kryofluid jeweils bei den Elektroden, d.h. innerhalb des jeweiligen von der Elektrode umschlossenen Volumens, freigesetzt, um die Elektrode gleichmäßig zu kühlen. Durch die gezielte Freisetzung des Kryofluids bei den Elektroden lässt sich lokal genau dort eine hohe Kühlleistung erzielen, wo eine hohe Wärmeabfuhr erforderlich ist, nämlich an den Elektroden. Andererseits wird eine zu starke Kühlung anderer Bereiche und somit ein Anfrieren von Gewebe an der Sonde ausgeschlossen. Damit lässt sich einerseits eine Leistungssteigerung der Ablationssonde oder andererseits eine weitere Durchmesserverminderung erzielen, ohne die Ablationsleistung zu kompromittieren.

Es können im Bereich der Elektroden an der Fluidlieferleitung jeweils eine oder auch mehrere seitliche Öffnungen vorgesehen sein. Vorzugsweise ist jedoch die Querschnittsfläche aller dem Fluidaustritt dienenden seitlichen Öffnungen insgesamt geringer als die Querschnittsfläche des inneren Kanals der Fluidlieferleitung. Durch diese Maßnahme wird der Druckabfall entlang der Länge der Fluidlieferleitung minimiert und im Wesentlichen auf die als Düsen dienenden seitlichen Öffnungen konzentriert. Der Kühleffekt wird auf diese Weise sehr gut auf dem Bereich der Elektroden lokalisiert. Insbesondere können die Elektroden unabhängig von ihrer axialen Wärmeleitfähigkeit wirksam gekühlt werden.

Die Fluidlieferleitung kann endseitig mit einem Verschlussstück oder einem Pfropfen aus Klebstoff oder einem anderen geeigneten, mit der Fluidlieferleitung eine feste Verbindungen eingehenden Material gebildet sein. Die Fluidlieferleitung ist vorzugsweise ein dünner Kunststoffschlauch, beispielsweise aus PE, PET oder PEEK oder einem anderen geeigneten Material mit geringem Elastizitätsmodul. Die seitlichen Öffnungen sind vorzugsweise Laserbohrungen mit einem Durchmesser zwischen 50 µm und 150 µm vorzugsweise 85 µm bis 125 µm. Der Innendurchmesser der Fluidlieferleitung beträgt vorzugsweise 0,4 mm bis 0,7 mm, zum Beispiel 0,6 mm oder 0,57 mm. Die Fluidlieferleitung ist somit außerordentlich flexibel.

Das Verschlussstück zum Verschluss des die Elektroden tragenden Schlauchs kann aus einem Isolationsmaterial, beispielsweise Kunststoff oder Keramik oder auch aus Metall bestehen. Insbesondere kann es auch aus einer Kombination eines elektrisch isolierenden Materials mit einem elektrisch leitfähigen Material bestehen. Die elektrisch leitfähigen Bereiche des Verschlussstücks können zum Beispiel als Elektroden genutzt werden, um das Einstechen des Instruments in biologisches Gewebe zu unterstützen. Die elektrisch leitfähigen Teile des Verschlussstücks ragen vorzugsweise kaum oder nicht aus dem isolierenden Material heraus. Die am distalen Ende so gebildete Elektrode kann das Einstechen des Instruments in biologisches Gewebe unterstützen, ohne dieses lateral zu schädigen.

Vorzugsweise ist das Verschlussstück mit einem zugfesten Draht fest verbunden, der sich von dem Verschlussstück durch das Lumen der Ablationssonde bis zu deren proximalen Ende erstreckt und dort verankert ist. Der Draht dient der Aufnahme von Zugkräften beim Entfernen des Instruments aus einem Patienten, insbesondere aus seinem Gewebe. Der Draht entlastet den Schlauch von Zugkräften, sodass das Instrument in jedem Fall, auch bei Behandlungsproblemen, beispielsweise infolge einer Anhaftung der Ablationselektroden am Gewebe, sicher geborgen werden kann. Der Zugdraht weist vorzugsweise eine hohe Rückstellkraft nach einer durch äußere Kraft induzierten Verformung, z.B. durch Abwinkelung des Zugangssystems, auf.

Bei der erfindungsgemäßen Ablationssonde ist die Fluidlieferleitung wenigstens an einer Stelle mit dem Zugdraht verbunden. Vorzugsweise ist die Fluidlieferleitung dabei zwischen den Elektroden und somit den (optional) mehrfachen Düsen mit dem Zugdraht verbunden. Alternativ kann sie aber auch an anderen, vorzugsweise außerhalb der Elektroden liegenden Bereichen, mit dem Zugdraht verbunden sein. Dadurch wird eine axiale Fehlpositionierung der seitlichen Öffnungen der Fluidlieferleitung in Bezug auf die Elektroden bei Biegung oder Abwinkelung des Instruments verhindert. Dies wiederum erlaubt eine sichere und verlässliche Handhabung und die Erzielung enger Biegeradien ohne Funktionsbeeinträchtigung.

Die Abwinkelung des Instruments wird auch dadurch unterstützt, dass die Elektroden flexibel ausgebildet sind. Sie können beispielsweise durch einen schraubenförmig gewundenen Draht oder durch schraubenförmig geschlitzte Hülsen oder dergleichen gebildet sein. Andere abwinkelbare geschlitzte Strukturen, wie beispielsweise mit halbkreisförmigen Schlitzen versehene Hülsen, können ebenso gut Anwendung finden. Zudem erlaubt die Funktionstrennung in Zugdraht zur Zugkraftaufnahme und Röhrengeometrie zur Fluidleitung im Ergebnis die Erzielung einer geringeren Biegesteifigkeit im Vergleich zu einem Fluidleiter mit ausreichender Wandstärke zur Fluiddruckaufnahme und Materialfestigkeit zur Zugkraftaufnahme. Bei der erfindungsgemäßen Gestaltung kann ein (Voll-)Material mit hoher Zugfestigkeit und einer Geometrie mit geringem Flächenmoment 2. Grades gewählt werden, um die Zugkraft aufzunehmen. Dieses wird mit einem Fluidleiter aus einem Material mit geringerer Festigkeit (das ausreichend ist, um dem Fluidruck standzuhalten) und hohem Innenquerschnitt kombiniert. Das hohe Flächenmoment 2. Grades bleibt aber wegen der hohen Materialnachgiebigkeit unschädlich.

Weitere Einzelheiten vorteilhafter Ausführungsformen der Erfindung sind Gegenstand von Unteransprüchen. In der Zeichnung ist ein Ausführungsbeispiel der Erfindung veranschaulicht. Es zeigen:
Figur 1 die erfindungsgemäße Ablationssonde, angeschlossen an ein speisendes Gerät, in perspektivischer Prinzipdarstellung,
Figur 2 das distale Ende der Ablationssonde, in Längsrichtung geschnitten mit Blick in sein Lumen,
Figur 3 ein Endstück für die Ablationssonde nach Figur 1 oder 2, in perspektivischer Darstellung,
Figur 4 das Endstück nach Figur 3, in teilweise aufgeschnittener Seitenansicht,
Figur5 das distale Ende des in dem Lumen der Ablationssonde angeordneten Fluidlieferschlauchs, in perspektivischer Ansicht.

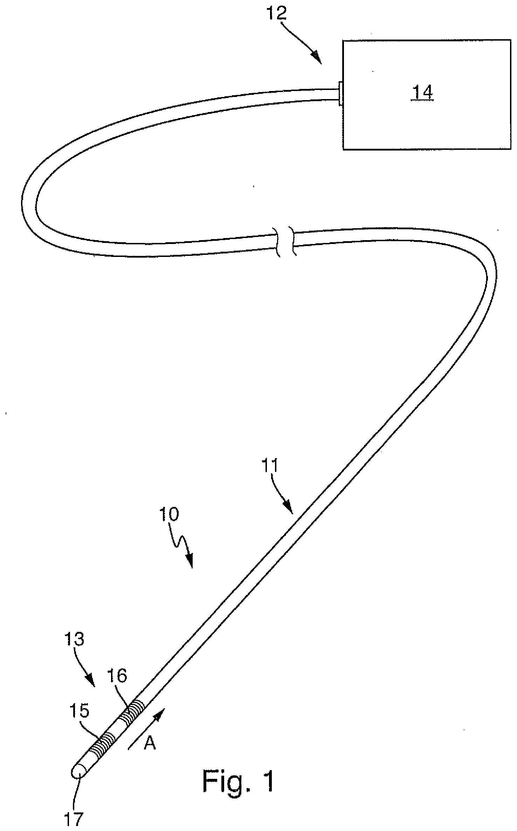

Figur 1 veranschaulicht eine Ablationssonde 10, wie sie für den endoskopischen, bspw. den bronchoskopischen Einsatz, beispielsweise zur Devitalisierung von Lungentumoren, aber auch zu anderen Zwecken einsetzbar ist. Die Ablationssonde 10 umfasst einen länglichen flexiblen Schlauch 11, der sich von einem proximalen Ende 12 bis zu dem distalen Ende 13 der Ablationssonde 10 erstreckt. An dem proximalen Ende 12 sind ein oder mehrere Stecker oder eine andere geeignete Anschlusseinrichtung vorgesehen, um die Ablationssonde 11 mit einem speisenden Gerät 14 zu verbinden.

Der Schlauch 11 ist vorzugsweise ein flexibler Kunststoffschlauch, der aus einem geeigneten bioverträglichen Kunststoff, beispielsweise PE, PET, PEEK oder ähnlichem gebildet ist. Während seine Länge mehrere Meter betragen kann, liegt der Durchmesser im Bereich von 1 mm bis 3 mm. Anderer Abmessungen sind je nach Anwendungsfall möglich und zweckmäßig.

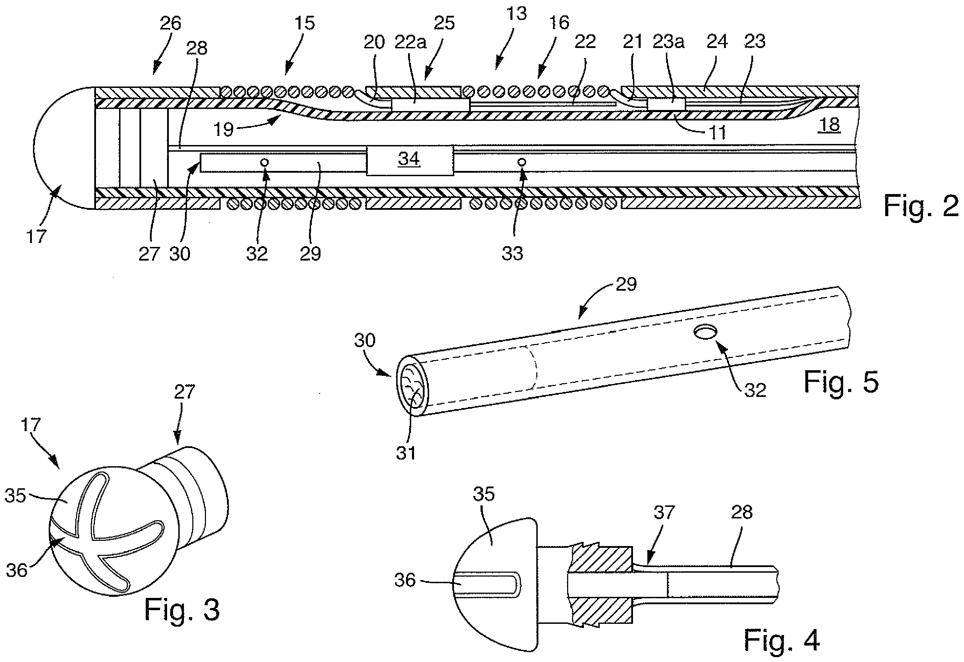

Das proximale Ende 13 der Ablationssonde 10 weist eine erste Elektrode 15 und, zumindest vorzugsweise, eine zweite Elektrode 16 auf, die in Axialrichtung A voneinander beabstandet sind. Es können bedarfsweise weitere bestrombare Elektroden vorgesehen sein. Das Instrument kann auch nur eine einzelne Elektrode aufweisen und als monopolares Instrment ausgelegt sein. Der Schlauch 11 ist an seinem distalen Ende außerdem mit einem Verschlussstück 17 versehen, das sein inneres aus Figur 2 hervorgehendes Lumen 18 distal abschließt.

Der Schlauch 11 umschließt dieses Lumen und ist dabei vorzugsweise lückenlos und unterbrechungsfrei ausgebildet. Seine Wandung weist keine Unterbrechungen oder Durchbrüche auf. Die Elektroden 15, 16 sind auf dem Schlauch 11 insbesondere in einem Bereich angeordnet, in dem in die Wandung des Schlauchs 11 eine längliche Vertiefung, z.B. Rinne 19 eingeprägt ist. In dieser liegen Anschlüsse 20, 21 der Elektroden 15, 16. Von den Anschlüssen 20, 21 erstecken sich elektrische Leitungen 22, 23 außen an dem Schlauch 11 entlang in proximaler Richtung zu dem Ende 12 und sind dort mit einem Stecker oder einer sonstigen Anschlusseinrichtung für das Gerät 14 verbunden. Die Leitungen 22, 23 sind mit den Anschlüssen 20, 21 über geeignete Anschlussmittel, z.B. Crimp-Hüsen 22a, 23a verbunden. Diese können, wie in Figur 2 dargestellt, jeweils in unmittelbarerer Nachbarschaft zu der angeschlossenen Elektrode 15, 16 oder alternativ beide proximal zu der proximalen Elektrode 16 angeordnet sein.

Die Leitungen 22, 23 erstrecken sich durch einen Spalt zwischen dem Schlauch 11 und einem Überzugsschlauch 24, der vorzugsweise aus einem sehr flexiblen, gleitfähigen Kunststoff gebildet ist. Zwischen den Elektroden 15, 16 kann ein hülsenförmiger Isolator 25 angeordnet sein, der zwischen den Elektroden 15, 16 als Distanzhalter dient. Außerdem kann zwischen der distalen Elektrode 15 und dem Verschlussstück 17 ein weiterer Isolator 26 angeordnet sein.

Das Verschlussstück 17 ist an seiner distalen Außenfläche vorzugsweise gerundet. Zum Beispiel kann es halbkugelförmig ausgebildet sein. Von dem halbkugelförmigen Kopf erstreckt sich ein Schaft 27 in das Lumen, wobei der Schaft 27 formschlüssig und/oder mittels Klebstoff oder sonstiger geeigneter Befestigungsmittel an dem Schlauch 11 gesichert ist. Außerdem ist das Verschlussstück 17 vorzugsweise mit einem Draht 28 verbunden, der sich von dem Verschlussstück ausgehend bis zu dem beispielweise am proximalen Ende 12 der Ablationssonde 10 angeordneten kraftübertragenden Teil der Ablationssonde 10 erstreckt, um Zugkräfte von dem proximalen Ende sicher auf das distale Ende 13 zu übertragen. Der kraftübertragende Teil der Ablationssonde 10 kann auch im Verlauf der Länge der Ablationssonde 10 angeordnet sein.

In dem Lumen 18 ist eine Fluidlieferleitung 29 angeordnet, die als dünner Kunststoffschlauch, gegebenenfalls aber auch als Metallkapillare ausgebildet sein kann. Die Fluidlieferleitung dient der Freisetzung von Kühlfluid, das die Elektroden 15, 16 kühlt und dann durch das Kühllumen 18 zu dem Gerät 14 zurückströmt. Es kann dort oder an dem proximalen Ende 12 der Ablationssonde 10 ins Freie entlassen oder auch aufgefangen und recycelt werden.

Die Fluidlieferleitung 29 ist an ihrem distalen Ende 30 geschlossen. Beispielsweise kann dazu ausgehärteter Klebstoff 31 (Figur 5) dienen, der als Pfropfen in dem distalen Endabschnitt der Fluidlieferleitung 29 sitzt.

Die Fluidlieferleitung 29 weist eine erste, als Austrittsdüse dienende seitliche Öffnung 32 und eine zweite, ebenfalls als Austrittsdüse dienende seitliche Öffnung 33 auf. Die erste Öffnung 32 dient der Kühlung der ersten Elektrode 15 und ist entsprechend axial in Registrierung mit dieser (d.h. innerhalb des von der ersten Elektrode 15 umschlossenen Raums) angeordnet. Vorzugsweise ist die Öffnung 32 dabei etwas weiter distal als die Mitte der Elektrode 15 angeordnet. Die zweite seitliche Öffnung 33 ist innerhalb des von der zweiten Elektrode 16 umschlossenen Raums zugeordnet. Sie ist dazu in Registrierung mit der zweiten Elektrode 16 angeordnet und kann etwas distal zu der Mitte der Elektrode 16 platziert sein.

Die Öffnungen 32, 33 sind ansonsten im Wesentlichen gleich ausgebildet. Sie können wie dargestellt in der gleichen Radialrichtung oder auch in Umfangsrichtung gegeneinander versetzt angeordnet oder in ihrem Querschnitt unterschiedlich sein.

Die nachfolgend in Verbindung mit der Figur 2 und 5 gegebene Erläuterung und Beschreibung der Öffnung 32 gilt ansonsten entsprechend für die Öffnung 33.

Während der Innendurchmesser der Fluidlieferleitung 29 im Bereich von etwa einem halben Millimeter liegen kann, beträgt der Durchmesser der vorzugsweise durch Laserbohren hergestellten Öffnung 32 lediglich etwa ein Zehntel Millimeter. Damit ist der von allen Öffnungen 32, 33 zusammen gebildeten Düsenquerschnitt deutlich geringer als der freie Strömungsquerschnitt (Querschnittsfläche) des Innendurchmessers und somit des Lumens der Fluidlieferleitung 29.

Im vorgestellten Ausführungsbeispiel ist jeder Elektrode 15, 16, unabhängig von deren Anzahl, jeweils eine Düsenöffnung 32, 33 individuell zugeordnet. Es ist jedoch auch möglich, jeder Elektrode 15, 16 zwei oder mehrere Öffnungen zuzuordnen, die axial und/oder in Umfangsrichtung gegeneinander versetzt sind. Damit ist auch bei besonders langen Elektroden 15, 16 eine sichere und gleichmäßige Kühlung derselben möglich.

Um die sichere axiale Relativpositionierung der Öffnungen 32, 33 bezüglich der Elektroden 15, 16 sicherzustellen, ist die Fluidlieferleitung 29 an ihrem distalen Ende axial fixiert. Dazu kann sie mit dem Draht 28 verbunden sein, der zum Beispiel aus einem Federstahl oder einem anderen besonders zugfesten Material, welches vorzugsweise eine hohe Rückstellneigung nach Verformung aufweist, wie zum Beispiel Nitinol hergestellt ist. Auch ist die Verwendung eines Kunststoffdrahts möglich.

Zur Befestigung der Fluidlieferleitung 29 an dem Draht 28 können diese zum Beispiel lokal an lediglich einer Stelle oder auch an mehreren Stellen miteinander verbunden werden. Insbesondere kann die Verbindung zwischen den Elektroden 15, 16 und somit zwischen den Öffnungen 32, 33 angeordnet sein. Die Verbindung zwischen dem Draht 28 und der Fluidlieferleitung 29 kann beispielsweise durch ein Klemmmittel 34 gebildet sein, das die Fluidlieferleitung 29 und den Draht 28 reibschlüssig aneinander hält. Das Klemmmittel 34 kann z.B. in Gestalt eines Schrumpfschlauchs 34 ausgebildet sein, durch den sich sowohl der Draht 28 als auch die Fluidlieferleitung 29 erstreckt und der den Draht 28 und die Fluidlieferleitung 29 gegeneinander klemmt. Anstelle des Schrumpfschlauchs kann auch ein anderes geeignetes Verbindungsmittel beispielsweise eine Crimphülse aus Metall, eine Klebeverbindung, eine Verbindung durch geschmolzenen und ausgehärteten Kunststoff, eine Federklemme oder ähnliches dienen.

Außerdem ist es möglich, zusätzlich oder anstelle der Verbindung zwischen dem Draht 28 und der Fluidlieferleitung 29 eine Verbindung zwischen dem distalen Ende der Fluidlieferleitung 29 und dem Verschlussstück 17 vorzusehen. Dazu können beispielsweise eine Klebeverbindung, eine Crimpverbindung, eine Quetschverbindung oder dergleichen vorgesehen sein. Beispielsweise kann das geschlossene Ende des Fluidlieferschlauchs 29 in eine Bohrung des Schafts 27 eingeklebt sein. Wesentlich ist aber bei allen Ausführungsformen, dass der Fluidlieferschlauch 29 insbesondere im Bereich seines distalen Endes bezüglich der Elektroden 15, 16 axial unbeweglich fixiert ist. Auf diese Weise können Fehlpositionierung des Fluidlieferschlauchs und seiner seitlichen Öffnungen 32, 33 infolge von Dehnung oder Biegung des Schlauchs 11 beim Endoskopischen Einsatz am Patienten vermieden werden.

Das Endstück 17 ist aus den Figuren 3 und 4 gesondert ersichtlich. Es kann vollständig aus einem isolierenden Material, wie zum Beispiel Kunststoff oder Keramik, oder auch vollständig aus Metall, beispielsweise aus Wolframkarbid ausgebildet sein. In den Figuren 3 und 4 ist jedoch eine besondere Ausführungsform veranschaulicht, bei der das Endstück 17 aus einem Metallkörper 36 und einem Isolator 35 gebildet ist. Der Metallkörper 36 liegt vorzugsweise an der gerundeten Endfläche des Verschlussstücks 17 zutage, wobei es wenig oder nicht über die gerundete distale Endfläche des Verschlussstücks 17 vorsteht. Der Isolator 35 kann distal auch etwas über den Metallkörper 36 vorstehen. Der Metallkörper 36 kann dabei beispielsweise, wie in Figur 3 dargestellt, an seinem distalen Ende kreuzförmig ausgebildet sein. Auch andere Formgebungen, wie beispielsweise ein einfacher Bogen oder ein drei- oder mehrarmiger Stern, sind möglich. Der Metallkörper 36 erstreckt sich durch den Isolator 35 und ragt, wie Figur 4 zeigt, an dem proximalen Schaftende heraus. Es kann dort zum Beispiel mittels einer Schweißverbindung 37 zugfest mit dem Draht 28 verbunden sein. Alternativ kann das Endstück 17 auch durch einen Metallkörper gebildet sein, auf den der Isolator in Gestalt eine Beschichtung aus Isolierstoff aufgebracht ist.

Der die Zugkräfte aufnehmende Draht 28 kann an dem proximalen Ende 12 mit einer elektrischen Verbindungseinrichtung vorgesehen sein, um von dem Gerät 14 mit Spannung und Strom versorgt zu werden. Dadurch wird das Metallinlay 36 zum Beispiel beim Einstechen der Ablationssonde 10 in biologisches Gewebe als Schneidelektrode wirksam.

Die insoweit beschriebene Ablationssonde 10 arbeitet wie folgt:

Die Ablationssonde 10 wird allein oder über ein entsprechendes Zugangsinstrument, beispielsweise ein Endoskop, oder, im Falle der Ablation eines Lungentumors, ein Bronchoskop, in den Bronchialbaum des Patienten eingeführt. Die Ablationssonde 10 wird dann in distaler Richtung aus dem Endoskop heraus weiter in distaler Richtung zu dem behandlungsbedürftigen Gewebe, beispielsweise dem Lungentumor, geschoben und in diesen eingestochen. Dies kann rein mechanisch oder, wenn eine distale Elektrode beispielsweise in Gestalt des Inlays 36 vorhanden ist, mit elektrischer Unterstützung erfolgen. Dazu wird die von dem Inlay 36 gebildete Elektrode aktiviert, indem ihm über den Draht 28 elektrische Energie zugeführt wird. Die Ablationssonde 10 wird dann soweit in den Tumor eingestochen, bis beide Elektroden 15, 16 innerhalb des Tumors platziert sind. Die distale Elektrode 36 ist danach inaktiv.

Es werden nun die Elektroden 15 und 16 mit Behandlungsspannung beaufschlagt und mit Behandlungsstrom versorgt. Zugleich oder kurz danach wird die Fluidzufuhrleitung 29 mit Kühlfluid beaufschlagt, sodass dieses aus den Öffnungen 32, 33 austritt. Das Kühlfluid, beispielsweise Kohlendioxid, steht in der Fluidlieferleitung mit einem hohen Druck von einigen 10 Bar (Zum Beispiel 65 Bar) an und tritt durch die Öffnungen 32, 33 in das Lumen 18 über, in dem ein geringerer Druck von allenfalls wenigen Bar herrscht. Die Öffnungen 32, 33 dienen dabei als Drosselöffnungen, wobei durch den Joule-Thomson-Effekt Kälte erzeugt wird. Durch die feste Registrierung, d.h. axiale Ausrichtung der Öffnungen 32, 33 in Bezug auf die Elektroden 15, 16 wird die Kühlwirkung insbesondere etwa mittig zu den Elektroden 15, 16 erzeugt, sodass eine weitgehend gleichmäßige Kühlung jeder Elektrode 15, 16 sichergestellt ist. Damit wird eine zu große Erwärmung der Elektroden 15, 16 und somit ein Austrocknen des anliegenden Gewebes auch dann vermieden, wenn der Durchmesser der Ablationssonde 10 im Wege der Miniaturisierung auf sehr geringe Werte von beispielsweise 2,3 mm oder weniger verringert wird, was zu hohen Stromdichten an den Elektroden 15, 16 führt. Mit dem erfindungsgemäßen Konzept ist jedoch eine Verbesserung und Vergleichmäßigung der Elektrodenkühlung zu bewirken, wodurch die Qualität der Ablationsbehandlung gefördert wird.

Eine erfindungsgemäße Ablationssonde 10 weist wenigstens eine Elektrode 15 und/oder 16 auf, die auf einem Schlauch 11 gehalten ist. Endseitig ist die Ablationssonde 10 mit einem Verschlussstück 17 versehen, von dem ausgehend sich ein Draht 28 über die gesamte Länge des Schlauchs 11 durch diesen erstreckt. An dem Draht 28 ist eine Fluidzulieferleitung 29 befestigt, die seitliche Öffnungen 32, 33 zur Kühlung der Elektroden 15, 16 aufweist und die endseitig geschlossen ist. Durch die Fixierung der Fluidzulieferleitung 29 an dem Draht 28 wird eine axiale Ausrichtung der Öffnungen 32, 33 relativ zu den Elektroden 15, 16 erreicht und eine Fehlpositionierung vermieden, die ansonsten zur Verungleichmäßigung der Kühlung der Elektroden 15, 16 führen könnte.

### Bezugszeichen:

- 10: Ablationssonde
- 11: Schlauch
- 12: proximales Ende der Ablationssonde 10
- 13: distales Ende der Ablationssonde 10
- 14: Gerät
- 15: erste Elektrode
- 16: zweite Elektrode
- 17: Verschlussstück
- 18: Lumen des Schlauchs 11
- 19: Rinne
- 20, 21: Anschlüsse der Elektroden 15, 16
- 22, 23: Leitungen
- 22a, 23a: Crimp-Hülsen
- 24: Überzugschlauch
- 25, 26: Isolator
- 27: Schaft
- 28: Zugdraht
- 29: Fluidlieferleitung
- 30: distales Ende der Fluidlieferleitung
- 31: Klebstoff
- 32: erste seitliche Öffnung / Düsenöffnung
- 33: zweite seitliche Öffnung / Düsenöffnung
- 34: Klemmmittel / Schrumpfschlauch
- 35: Isolator
- 36: Metallkörper
- 37: Schweißverbindung

## Patentansprüche

1. Ablationssonde (10), insbesondere zur HF-Ablation, mit einem flexiblen Schlauch (11), auf dem mindestens eine erste Elektrode (15, 16) angeordnet ist, und die ein distal geschlossenes Lumen (18) aufweist,
mit einer in dem Lumen (18) angeordneten Fluidlieferleitung (29), die wenigstens eine erste seitliche Öffnung (32) im Bereich der ersten Elektrode (15) und wenigstens eine zweite seitliche Öffnung (33) im Bereich der zweiten Elektrode (16) aufweist.

2. Ablationssonde nach Anspruch 1, **dadurch gekennzeichnet, dass** die Fluidlieferleitung (29) distal geschlossen ist.

3. Ablationssonde nach einem der vorstehenden Ansprüche, **dadurch gekennzeichnet, dass** der Schlauch (11) distal mit einem Verschlussstück (17) versehen sind.

4. Ablationssonde nach Anspruch 3, **dadurch gekennzeichnet, dass** das Verschlussstück (17) aus einem Isolationsmaterial oder aus einem elektrisch leitfähigen Material besteht.

5. Ablationssonde nach Anspruch 3, **dadurch gekennzeichnet, dass** das Verschlussstück (17) aus einer Kombination aus einem elektrisch nicht leitfähigen und elektrisch leitfähigen Material besteht.

6. Ablationssonde nach Anspruch 5, **dadurch gekennzeichnet, dass** das Verschlussstück (17) aus einem Isolator (35) und einem Metallkörper (36) besteht, das an der distalen Endfläche frei liegt.

7. Ablationssonde nach Anspruch 6, **dadurch gekennzeichnet, dass** der Isolator (35) durch eine Beschichtung des Metallkörper (36) gebildet ist.

8. Ablationssonde nach Anspruch 3 oder 4, **dadurch gekennzeichnet, dass** das Verschlussstück (17) mit einem Draht (28) verbunden ist, der sich durch das Lumen (18) erstreckt.

9. Ablationssonde nach Anspruch 8, **dadurch gekennzeichnet, dass** die Fluidlieferleitung (29) an wenigstens einer Stelle mit dem Draht (28) verbunden ist.

10. Ablationssonde nach Anspruch 9, **dadurch gekennzeichnet, dass** die Kombination aus dem aus Vollmaterial bestehenden Draht (28) und der Fluidlieferleitung (29) bei gleicher Zugfestigkeit eine geringere Biegesteifigkeit aufweist als ein Rohr, welches die Zugkraftaufnahme und Fluidversorgung kombiniert.

11. Ablationssonde nach Anspruch 9 oder 10, **dadurch gekennzeichnet, dass** der Draht (28) und die Fluidlieferleitung (29) mittels eines Klemmmittels (34) miteinander verbunden sind.

12. Ablationssonde nach einem der vorstehenden Ansprüche, **dadurch gekennzeichnet, dass** die Elektroden (15, 16) flexibel ausgebildet sind.

13. Ablationssonde nach einem der vorstehenden Ansprüche, **dadurch gekennzeichnet, dass** der Schlauch (11) unterbrechungsfrei ausgebildet ist.

14. Ablationssonde nach einem der vorstehenden Ansprüche, **dadurch gekennzeichnet, dass** die Fluidlieferleitung (29) ein Kunststoffschlauch ist.

15. Ablationssonde nach einem der vorstehenden Ansprüche, **dadurch gekennzeichnet, dass** die erste und die zweite Öffnung (32, 33) Laserbohrungen sind.

16. Verfahren zur Tumorablation mit einer Ablationssonde nach einem der Ansprüche 1 bis 15, mit folgenden Schritten:
Einstechen der Ablationssonde (10) in Gewebe, das einen behandlungsbedürftigen Bereich, z.B. einen Tumor, enthält,
Positionieren der mindestens einen Elektrode (15) in dem behandlungsbedürtigen Gewebe,
Bestromung der Elektrode (15) und zugleich Kühlung der Elektrode (15) durch Kälteerzeugung mittels eines gasförmigen Fluids, das über die Fluidlieferleitung in ein von der Elektrode umfasstes Lumen (18) geleitet und über die Drosselöffnung (32) in das Lumen (18) entlassen wird, um unter Nutzung des Joule-Thomson-Effekts_Kälte zu erzeugen,
Rückziehen der Ablationssonde (10) durch Übertragung von Zugkraft über den Zugdraht (28) auf das Verschlussstück (17).

17. Verfahren nach Anspruch 16, **dadurch gekennzeichnet, dass** das beim Einstechen der Ablationssonde (10) der Metallkörper (36) mit einer elektrischen Spannung beaufschlagt wird, um das distal vor dem Metallkörper (36) liegende Gewebe zu schneiden und so das einstechen zu erleichtern.
